**Europäisches Patentamt**

**European Patent Office**

**Office européen des brevets**

(11) Publication number : **0 437 956 B1**

## EUROPEAN PATENT SPECIFICATION

(45) Date of publication of patent specification :
**01.06.94 Bulletin 94/22**

(21) Application number : **90313908.7**

(22) Date of filing : **19.12.90**

(51) Int. Cl.⁵ : **A61K 7/50**

(54) **Cosmetic composition.**

(30) Priority : **21.12.89 GB 8928903**

(43) Date of publication of application :
**24.07.91 Bulletin 91/30**

(45) Publication of the grant of the patent :
**01.06.94 Bulletin 94/22**

(84) Designated Contracting States :
**AT BE CH DE DK ES FR GB GR IT LI NL SE**

(56) References cited :
**EP-A- 0 213 827**
**EP-A- 0 257 336**
**EP-A- 0 307 086**
**FR-A- 1 501 837**

(73) Proprietor : **UNILEVER PLC**
**Unilever House**
**Blackfriars**
**P.O. Box 68**
**London EC4P 4BQ (GB)**
㊗ **GB**
Proprietor : **UNILEVER N.V.**
**Weena 455**
**NL-3013 AL Rotterdam (NL)**
㊗ **BE CH DE DK ES FR GR IT LI NL SE AT**

(72) Inventor : **Rosser, David Arthur**
**Quarry Road East,**
**Bebington**
**Wirral, Merseyside L63 3JW (GB)**

(74) Representative : **Ford, Michael Frederick et al**
**MEWBURN ELLIS**
**York House**
**23 Kingsway**
**London WC2B 6HP (GB)**

## Description

FIELD OF THE INVENTION

The invention relates to a cleansing composition suitable for topical application to human skin, more particularly to a propellant-driven cleansing mousse composition for removal of make-up from the skin.

BACKGROUND AND PRIOR ART

The topical application to human skin, in particular to the face, has since time immemorial been and still is, an art form employed particularly by women, as part of a daily or periodic ritual or routine to embellish or beautify their appearance in the eyes of the beholder and/or to enhance confidence, to enable them more readily to face each day. Topical application of make-up, particularly to exposed areas of the skin, can also provide some protection from the elements, such as the sun, the wind and the rain, where otherwise the skin damage or accelerated skin ageing can occur.

Make-up, once applied to the skin, has conventionally only a limited life, and must be removed from time to time and replenished anew. To habitual make-up users, this is a daily or twice daily activity.

The removal of make-up, particularly waxed based make-up such as lipstick and mascara, presents a special problem in that it can adhere strongly to the skin and can resist ordinary washing with soap and water, or with mild detergent products especially formulated for use on delicate skin areas, such as the face. Scrubbing of the skin to remove make-up can be successful, but damage to the underlying sensitive skin can result.

Oil-based cleansing products such as 'cold cream' have been recommended for cleaning make-up from the skin, but the resultant oil residue consisting of a mixture of solubilised make-up and excess cleanser is difficult to remove either by wiping off or by rinsing with water.

Mousses delivered from a pressurised container offer a useful alternative cleansing product in that they are convenient to apply and are generally easy to remove after use.

One such product currently available on the UK market is Johnsons Baby Oil Mousse, which is marketed primarily as a moisturising product, but which can be used with only moderate success to remove lipstick. This product is described in EP 0 307 086, and is stated to contain mineral oil, a $C_{16-18}$ fatty alcohol ethoxylate, having an HLB value of less than 10 as well as propellant and water.

A mild, skin-cleansing, non-foaming mousse-forming emulsion is described by Procter & Gamble in EP 0 213 827. The mousse comprises a nonionic surfactant, such as an ethoxylated nonionic surfactant or a partially esterified polyol, an emollient such as a mineral oil or vegetable oil, a moisturiser such as glycerin or sorbitol and a water soluble gaseous propellant such as carbon dioxide and nitrogen. The composition is preferably substantially free from water-insoluble propellants such as hydrocarbons. The composition which is exemplified contains minor amounts of esters.

SUMMARY OF THE INVENTION

Attempts to employ Johnsons Baby Oil Mousse, as well as other body moisturising cleansing products such as 'cold cream', in the removal of tenacious make-up, have met with only partial success, and accordingly, there remains a problem of complete removal of make-up without resort to solvents or physical abrasion that might cause damage to the skin.

We have now discovered that by use of an oil which is an ester together with a nonionic emulsifier and water, the emulsion being pressurised with a water insoluble propellant, a mousse-generating emulsion is obtained which has excellent make-up removal properties, and can be easily removed from the skin by wiping or by rinsing with water.

DEFINITION OF THE INVENTION

Accordingly, the invention provides a cleansing composition suitable for topical application to human skin to remove make-up, the composition comprising:

a. from 90 to 99.5% by weight of a concentrate comprising

i. from 10 to 40% by weight of an oil having at least one ester group, in which the alkanoate moiety has from 8 to 22 carbon atoms;

ii. from 2 to 20% by weight of a nonionic emulsifier having an average HLB value of from 5 to 14, said emulsifier comprising an alkyl or alkaryl moiety having from 9 to 15 carbon atoms and from 2 to 10 ethylene oxide units; and

iii. the balance water; and

b. from 2 to 10 % by weight of a propellant.

DISCLOSURE OF THE INVENTION

The cleansing composition according to the invention comprises a concentrate, including at least a special oil, a special emulsifier and water, and a propellant which is filled together with the concentrate into pressurised container to provide the cleansing composition of the invention, which can then be dispensed as a mousse.

The Ester Oil

The cleansing composition according to the invention comprises an oil having at least one ester group, in which the alkanoate moiety has from 8 to 22 carbon atoms.

By "oil" we mean a cosmetically acceptable substantially water-immiscible liquid.

The oil may in particular be a triglyceride oil and furthermore may preferably have branched chain alkanoate moieties.

Examples of oils for use in the composition according to the invention include:

saturated or unsaturated, straight or branched chain $C_{8-22}$ alkane esters, for example

isopropylmyristate (e.g. ESTOL 1514, ex Unichema) hexyl laurate (e.g. CETIOL A, ex Henkel) methyl laurate (e.g. ESTOL 1502, ex Unichema) 2-ethylhexyl palmitate (e.g. GLYCO 0-3000, ex Glyco)

2-octyldodecyl myristate (e.g. MOD, ex Nippon Oils & Fats)

propyleneglycol dicaprylate/caproate (e.g. ESTOL 1526, ex Unichema)

isopropylpalmitate (e.g. ESTOL 1517, ex Unichema) decyloleate (e.g. CETIOL V, ex Henkel)

triglycerides such as

glyceryl tri-caprylate/caprate (e.g. ESTOL 1527, ex Unichema)

glyceryl tri-isostearate (e.g. PRISORINE, ex Unichema)

glyceryl tri(2-ethylhexanoate) (e.g. MYRITOL GTEH, ex Henkel).

The amount of oil present in the composition of the invention is from 10 to 40%, preferably 20 to 35% by weight.

Compositions containing less than 10% by weight of oil tend to be poor at removing make-up from human skin, while those containing more than 40% by weight of the oil can leave the skin in a greasy state after use and their efficacy at removing make-up is not further enhanced.

The Nonionic Emulsifier

The cleansing composition according to the invention also comprises a nonionic emulsifier having an average HLB value of from 5 to 14, said emulsifier comprising an alkyl or alkaryl moiety having from 9 to 15 carbon atoms and from 2 to 10 ethylene oxide units.

Preferably, the nonionic emulsifier had an average HLB value of from 10 to 14.

Examples of nonionic emulsifiers include:

Laureth 2 (e.g. EMPILAN KB2, ex Albright & Wilson)

Laureth 7 (e.g. MARLIPAL MG, ex Huls)

PEG 8 laurate (e.g. CLITHROL 4ML, ex Croda)

Pareth 25.7 (e.g. SYNPERONIC A7, ex ICI)

Pareth 91-6 (e.g. SYNPERONIC 91/6, ex ICI)

Nonoxynol 7 (e.g. SYNPERONIC NP7, ex ICI)

The amount of nonionic emulsifier present in the composition of the invention is from 2 to 20% by weight.

The amount of ester oil will, in many compositions of this invention, be greater than the quantity of the emulsifier and also greater than the quantity of all surfactants present, including the emulsifier. The quantity of emulsifier may lie in a range from 2 to 10% by weight.

Water

The cleansing composition according to the invention also comprises water. This may be present in an amount from 20% to 86% preferably from 50 to 86% by weight, yet more preferably 50 to 70% by weight.

The Propellant

The cleansing composition according to the invention also comprises a substantially water-insoluble propellant.

Examples of suitable propellants include:

    propane,

    iso-butane,

    n-butane,

    mixtures of these three propellants, such as CAP 30 (ex Calor)

    dimethylether,

    hydrofluorocarbon, such as HFA 152a and 134a,

    chlorofluorocarbons, such as Propellants 11, 12, 114 and 22.

Mixtures of water-insoluble propellants can also be used.

The amount of propellant present in the composition of the invention is from 2 to 10%, preferably from 3 to 6% by weight.

OTHER INGREDIENTS

Hydrocarbon oil

The cleansing composition according to the invention can also optionally comprise a hydrocarbon oil as a supplement to the oil having at least one ester group, which is an essential ingredient of the composition of the invention. The presence of a hydrocarbon oil can, accordingly, further enhance the cleansing properties of the cleansing composition.

Examples of mineral oils, when present, include:

    technical white oil (e.g. SIRIUS M85, ex Dalton)

    technical white oil (e.g. RUDOL, ex Witco)

    iso-paraffin (e.g. ISOPAR L, ex Exxon)

    polybutane (e.g. POLYSYNLANE, ex Nippon Oil & Fat)

The amount of hydrocarbon oil when present in the composition of the invention is usually up to 30%, preferably from 5 to 20% by weight of the composition.

Cosmetic adjuncts

The composition according to the invention can optionally comprise cosmetic adjuncts, examples of which are:

    preservatives, such as:

        p-hydroxybenzoate esters

        2-bromo-2-nitropropane-1, 3-diol

        salicylic acid

    antioxidants, such as:

        butylated hydroxy toluene

        butylated hydroxy anisole

        tocopherol

    skin conditioners, such as

        Polyquaternium 10

        PEG-7 glyceryl cocoate

    emulsion stabilisers (co-emulsifiers), such as:

        cetyl alcohol

        glyceryl mono/distearate

        tearic acid

    humectants, such as:

        glycerol

        propylene glycol

        dipropylene glycol

        sorbitol

        2-pyrrolidone-5-carboxylate

        polyethyleneglycol (e.g. PEG 200-600)

thickeners, such as
    carbomers
    xanthan gum
    hectorite
    fumed silica
plant extracts, such as
    Aloe vera
    cornflower
    witch hazel
    elderflower
    cucumber
germicides, such as
    triclosan
    cetrimide
colourants and perfumes.

Cosmetic adjuncts can form up to 50% by weight of the composition and can conveniently form the balance of the composition.

## Process for preparing the composition

The invention also provides a process for the preparation of a cleansing composition for topical application to skin which comprises the step of incorporating into the composition an oil having at least one ester group, a nonionic emulsifier and water, to form a concentrate which is then filled into pressurised containers together with a substantially water-insoluble propellant.

## Use of the composition

The emulsion according to the invention is intended primarily as a product for topical application to cleanse human skin, particularly to remove make-up from the face and other parts of the body.

In use, a small quantity of the composition, for example from 1 to 5 ml, is delivered as a mousse from an aerosol dispenser and then applied to the affected area of skin. If necessary, the mousse is then spread over and/or rubbed onto the skin using the hand or fingers or a suitable device, in order to effect a cleansing action.

The emulsified residue can then be removed by wiping off with a tissue or by rinsing with water.

## PRODUCT FORM AND PACKAGING

The topical skin cleansing composition of the invention can be formulated as a liquid having a viscosity usually of from 10 to 2,000 mPas, as measured with a Brookfield RVT viscometer using spindle 3 at 25°C. The composition can be packaged in a suitable pressurised container, from which it can be dispensed as a mousse.

The invention accordingly also provides a closed container containing the cosmetically acceptable cleansing composition as herein defined, the container being a pressurised container fitted with a closure incorporating a valve and actuater suitable for dispensing a mousse.

## Evidence to demonstrate ability of the composition in removing make-up from skin

### The Subjective Lipstick Removal Test

The subjective test was performed by human volunteers, to whose forearms was applied a lipstick mark in the shape of a 'cross'. An attempt was then made to remove the 'cross' by following a standard cleansing procedure using a variety of compositions, some in accordance with the invention and some outside the monopoly claimed.

### Materials

The lipstick chosen for this test was Cutex Lip Moist 040.
The cleansing products had the following formulation:

5

| Ingredients (concentrate) | % w/v |
|---|---|
| ester oil | 24 |
| emulsifier | 6 |
| water | 70 |

The concentrate was filled into aerosol cans and pressurised with CAP 30 to a level of 5% by weight of the emulsion.

Method

A lipstick mark, in the shape of a cross (3cm by 3cm) was applied to the left forearm of each right handed volunteer (or vice versa if left handed).

A 2cm diameter dose of mousse expelled from the aerosol can was applied directly to the lipstick cross and rubbed in for 10 seconds.

The arm was finally rinsed in luke-warm water and removal of the lipstick estimated subjectively as follows:
0 none removed
1 poor removal
2 satisfactory removal
3 complete removal

Results

The results of a series of cleansing tests are set out in the following table.

For each emulsifier the CTFA name is stated, together with the HLB value and the chain length i.e. the number of carbon atoms in the alkyl group of the emulsifier.

| Expt No. | Oil | Emulsifier (CTFA name) | HLB of Emulsifier | Chain Length | Removal Score |
|---|---|---|---|---|---|
| 1 | GTEH | Laureth 2 | 7.0 | 12 | 2 |
| 2 | GTEH | Laureth 7 | 13.0 | 12 | 3 |
| 3 | GTEH | Laureth 4 | 9.7 | 12 | 2 |
| 4 | GTEH | 3:1 Laureth 2 :Laureth 23 | | 12 | 2 |
| 5 | GTEH | Steareth 2 | 4.0 | 18 | 1 |
| 6 | GTEH | Oleth 2 | 4.9 | 18 | 1 |
| 7 | GTEH | Oleth 5 | 8.8 | 18 | 1 |
| 8 | GTEH | PEG 4/laurate | 4.8 | 12 | 1 |

| | | | | | |
|---|---|---|---|---|---|
| 9 | GTEH | PEG 8 laurate | 13.1 | 12 | 3 |
| 10 | GTEH | Pareth 23.2 | 6.5 | 12-13 | 2 |
| 11 | GTEH | Pareth 25.2 | 5.9 | 12-15 | 2 |
| 12 | GTEH | Pareth 25.3 | 7.8 | 12-15 | 2 |
| 13 | GTEH | Pareth 25-7 | 12.2 | 12-15 | 3 |
| 14 | GTEH | Pareth 91-2.5 | 8.2 | 9-11 | 2 |
| 15 | GTEH | Pareth 91-4 | 10.8 | 9-11 | 2 |
| 16 | GTEH | Pareth 91-6 | 11.8 | 9-11 | 3 |
| 17 | GTEH | Pareth 91-12 | 15.3 | 9-11 | 1 |
| 18 | GTEH | Pareth 91-20 | 16.9 | 9-11 | 1 |
| 19 | GTEH:MO#1* | Laureth 2 | 7.0 | 12 | 3 |
| 20 | GTEH:MO#2* | Laureth 2 | 7.0 | 12 | 3 |
| 21 | GTEH:MO#2* | PEG4 laurate | 4.8 | 12 | 2 |
| 22 | IPM | Laureth 7 | 13.0 | 12 | 3 |
| 23 | IPM | PEG 8 Laurate | 13.1 | 12 | 3 |
| 24 | IPM | Laureth 2 | ca 7 | 12 | 2 |
| 25 | DO | Laureth 2 | ca 7 | 12 | 2 |
| 26 | DO | Laureth 7 | ca 13 | 12 | 3 |
| 27 | DO | PEG 8 Laurate | 12.1 | 12 | 3 |

Note that:     GTEH is glyceryl tri-(2-ethylhexanoate)

MO#1 is mineral oil 85 SUS

MO#2 is mineral oil 350 SUS

IPM is iso-propyl myristate

DO is decyl oleate

*weight ratio of 1:1

Ingredient Sources

Oils

| Glyceryl tri-(2 ethylhexanoate) | Myritol GTEH ex Henkel (Japan) |
|---|---|
| Mineral Oil 85 SUS | Sirius M85 ex Dalton |
| Mineral Oil 350 SUS | Sirius M350 ex Dalton |
| Isopropyl Myristate | Estol 1514 ex Unichema |
| Decyl Oleate | Cetiol V ex Henkel |

Emulsifiers

| Laureth 2 | Empilan KB2 ex Albright & Wilson |
|---|---|
| Laureth 7 | Marlipal MG ex Huls |
| Laureth 4 | Brij 30 ex Atlas/ICI |
| Laureth 23 | Brij 35 ex Atlas/ICI |
| Stearate 2 | Brij 72 ex Atlas/ICI |
| Oleth 2 | Brij 92 ex Atlas/ICI |
| Oleth 5 | Volpo N5 ex Croda |
| PEG 4 laurate | Clithrol 2ML ex Croda |
| PEG 8 laurate | Clithrol 4 ML ex Croda |
| Pareth 23.2 | Dobanol 23 EO ex Shell |
| Pareth 25.2 | Synperonic A2 ex ICI |
| Pareth 25.3 | Synperonic A3 ex ICI |
| Pareth 25.7 | Synperonic A7 ex ICI |
| Pareth 91-2.5 | Synperonic 91/2.5 ex ICI |
| Pareth 91-4 | Synperonic 91/4 ex ICI |
| Pareth 91-6 | Synperonic 91/6 ex ICI |
| Pareth 91-12 | Synperonic 91/12 ex ICI |
| Pareth 91-20 | Synperonic 91/20 ex ICI |

Conclusions

From these results, it is apparent that the most effective formulations for removing lipstick, in accordance with the Lipstick Removal Test are:

i. those that contain an emulsifier having an average HLB value of between about 12 and 13, (i.e. Experiments 2, 9, 13, 16, 20, 22, 26 and 27) and

ii. those that contain added hydrocarbon oil and an emulsifier having an average HLB value below 12 (i.e. Experiment 19).

It is also apparent that those formulations which are moderately effective in removing lipstick in accordance with this Test are:

i. those that contain an emulsifier having an average HLB value of between about 5 to 10, (i.e. Experiments 1, 3, 4, 10, 11, 12, 14, 15, 20, 24 and 25).

It is also apparent that those formulations which are poor in removing lipstick in accordance with this Test are:

i. those that contain an emulsifier having an average HLB value of below 5 (i.e. Experiments 5, 6 and 8); and

ii. those that contain an emulsifier having an average HLB value of above 14 (i.e. Experiments 12 and 18),

iii. those that contained an emulsifier having an average HLB value of greater than 5, but with an alkyl chain greater than $C_{15}$ (i.e. Experiment 7).

8

Overall, a preference can be seen for those formulations which
a. contain an emulsifier having an average HLB value of between 12 and 13; and
b. contain a hydrocarbon oil as a supplement to but not a replacement for an ester oil.

Examples

The invention is further illustrated by the following examples, which illustrate cleansing compositions according to the invention.

### Example 1

| Ingredients | % w/w |
|---|---|
| Glyceryl tri(2-ethylhexanoate) | 23 |
| Laureth 7 | 6 |
| Water | 64 |
| CAP 30 | 5 |
| Glycerol | 2 |
| Minor ingredients, including preservative and perfume | q.s. |

### Example 2

| | |
|---|---|
| Isopropyl myristate | 30 |
| PEG 8 laurate | 8 |
| Glycerol | 2 |
| Water | 50 |
| CAP 30 | 10 |
| Minor ingredients | q.v. |

## Example 3

| | |
|---|---|
| Decyl oleate | 12 |
| Technical white oil | 12 |
| Laureth 2 | 6 |
| Dipropylene glycol | 3 |
| Dimethyl ether | 5 |
| Water | 62 |

## Example 4

| | |
|---|---|
| Glyceryl tri-isostearate | 20 |
| Polybutene | 4 |
| Glycerol | 3 |
| CAP 30 | 4 |
| Dimethyl ether | 4 |
| Water | 58 |
| Pareth 91-6 | 7 |

## Example 5

| | |
|---|---|
| Propylene glycol di-caprylate/caprate | 23 |
| Pareth 25-7 | 8 |
| Propylene glycol | 2 |
| CAP 30 | 6 |
| Water | 61 |

Example 6

| | |
|---|---|
| Glyceryl tri(2-ethylhexanoate) | 34.0 |
| Laureth 7 | 6.0 |
| Cetearyl alcohol | 3.0 |
| Glycerol | 2.0 |
| CAP 30 | 5.0 |
| Water | 50.0 |
| Minor ingredients | q.v. |

Example 7

| | |
|---|---|
| Glyceryl tri(2-ethylhexanoate) | 12.0 |
| Technical white oil | 12.0 |
| Xanthan gum | 0.5 |
| Laureth 7 | 6.0 |
| Glycerol | 2.0 |
| CAP 30 | 5.0 |
| Water | 62.5 |
| Minor ingredients | q.v. |

Quantitative Testing

The compositions of some of the foregoing examples, and some other cleansers, were tested by the following test which assesses removal of lipstick from a panellist's forearm.

The lipstick chosen for this test was Rimmel Truly Red Lipstick.

An area of approximately 3cm by 5cm on the panellists forearm was wiped with alcohol.

The reflectance of the clean skin ($R_S$) was measured using a Minolta chromameter CR100.

Approximately 0.2g of the lipstick was applied to the clean area of skin and the reflectance ($R_L$) measured.

1 gram of the cleansing composition was applied to the test area, rubbed over the area for 20 seconds, and removed by rinsing with water (37°C) or wiping with tissue as appropriate for the cleansing composition concerned. Reflectance ($R_F$ of the skin area was measured.

Percentage lipstick removed was expressed as

$$100 - \frac{(R_F - R_S)}{(R_L - R_S)} \times 100\%$$

The test was carried out with six panellists for each composition. The results were averaged and are set out in the following table.

11

| Cleansing Composition | Average % Lipstick Removal |
|---|---|
| Pond's Cold Cream | 77 |
| Nivea Cleansing Milk | 63 |
| Johnsons Baby Oil Mousse | 77 |
| Example 1 | 91 |
| Example 3 | 92 |
| Example 4 | 91 |
| Example 5 | 96 |
| Example 6 | 92 |
| Example 7 | 84 |

The advantage achieved with compositions according to this invention is plain to see.

## Claims

1. A cleansing composition suitable for topical application to human skin to remove make-up, the composition comprising
   a. from 90 to 99.5% by weight of a concentrate comprising
      i. from 10 to 40% by weight of an oil having at least one ester group, in which the alkanoate moiety has from 8 to 22 carbon atoms;
      ii. from 2 to 20% by weight of a nonionic emulsifier having an average HLB value of from 5 to 14, said emulsifier comprising an alkyl or alkaryl moiety having from 9 to 15 carbon atoms, and from 2 to 10 ethylene oxide units;
      iii. water; and
   b. from 2 to 10% by weight of a propellant.

2. A composition according to claim 1, in which the oil is a triglyceride oil.

3. A composition according to claim 2, in which the triglyceride oil comprises one or more branched chain ester groups.

4. A composition according to claim 3, in which the triglyceride oil is glyceryl tri(2-ethylhexanoate).

5. A composition according to claim 1, in which the oil is isopropyl myristate.

6. A composition according to claim 1, in which the oil is decyl oleate.

7. A composition according to any preceding claim, in which the emulsifier has an HLB value of from 12 to 14.

8. A composition according to any preceding claim, in which the emulsifier is chosen from:
      Laureth® 2
      Laureth® 7
      PEG® 8 laurate
      Pareth® 25.7

Pareth® 91-6, and

Nonoxynol® 7.

9. A composition according to any preceding claim, which further comprises a hydrocarbon oil.

10. A composition according to any preceding claim, in which the propellant is CAP® 30.

11. A composition according to any preceding claim, which comprises
    a. from 85 to 95% by weight of a concentrate comprising:
        i. from 15 to 30% by weight of glyceryl tri(2-ethylhexanoate);
        ii. from 2 to 10% by weight of Laureth® 7, having an average HLB value of from 12.5 to 13.5; and
        iii. from 20 to 70% by weight of water; and
    b. from 3 to 10% by weight of CAP 30.


## Patentansprüche

1. Reinigende Zusammensetzung mit Eignung zur topischen Applikation auf menschliche Haut zur Entfernung von Make-up, umfassend,
    a. 90 bis 99,5 Gew.% eines Konzentrats umfassend
        i. 10 bis 40 Gew.% eines Öls mit mindestens einer Estergruppe, bei dem die Alkanoateinheit 8 bis 22 Kohlenstoffatome aufweist,
        ii. 2 bis 20 Gew.% eines nichtionischen Emulgators mit einem durchschnittlichen HLB-Wert von 5 bis 14, wobei der Emulgator eine Alkyl- oder Alkaryleinheit mit 9 bis 15 Kohlenstoffatomen und 2 bis 10 Ethylenoxideinheiten umfaßt, und
        iii. Wasser sowie
    b. 2 bis 10 Gew.% eines Treibmittels.

2. Zusammensetzung nach Anspruch 1, bei der es sich bei dem Öl um ein Triglyceridöl handelt.

3. Zusammensetzung nach Anspruch 2, bei der das Triglyceridöl eine oder mehrere verzweigtkettige Estergruppen umfaßt.

4. Zusammensetzung nach Anspruch 3, bei der das Triglyceridöl aus Glyceryltri(2-ethylhexanoat) besteht.

5. Zusammensetzung nach Anspruch 1, bei der das Öl aus Isopropylmyristat besteht.

6. Zusammensetzung nach Anspruch 1, bei der das Öl aus Decyloleat besteht.

7. Zusammensetzung nach einem der vorhergehenden Ansprüche, bei der der Emulgator einen HLB-Wert von 12 bis 14 aufweist.

8. Zusammensetzung nach einem der vorherigen Ansprüche, bei der der Emulgator aus
    Laureth® 2,
    Laureth® 7,
    PEG® 8 Laurat,
    Pareth® 25,7,
    Pareth® 91-6 und
    Nonoxynol® 7
    besteht.

9. Zusammensetzung nach einem der vorherigen Ansprüche, des weiteren umfassend ein Kohlenwasserstofföl.

10. Zusammensetzung nach einem der vorherigen Ansprüche, bei der das Treibmittel aus CAP® 30 besteht.

11. Zusammensetzung nach einem der vorherigen Ansprüche, umfassend
    a. 85 bis 95 Gew.% eines Konzentrats umfassend:
        i. 15 bis 30 Gew.% Glyceryltri(2-ethylhexanoat),
        ii. 2 bis 10 Gew.% Laureth 7 eines durchschnittlichen HLB-Werts von 12,5 bis 13,5 und

iii. 20 bis 70 Gew.% Wasser sowie

b. 3 bis 10 Gew.% CAP 30.

## Revendications

1. Composition nettoyante qui convient pour application locale à la peau afin d'effectuer un démaquillage, la composition comprenant :

    a. de 90 à 99,5% en poids d'un concentré comprenant :

    i. de 10 à 40% en poids d'une huile comportant au moins un groupe ester, dans lequel le fragment alcanoate contient de 8 à 22 atomes de carbone ;

    ii. de 2 à 20% en poids d'un émulsifiant non ionique ayant un indice d'amphipathie moyen de 5 à 14, ledit émulsifiant comprenant un fragment alkyle ou alcaryle contenant de 9 à 15 atomes de carbone et de 2 à 10 motifs d'oxyde d'éthylène ; et

    iii. de l'eau ;

    et

    b. de 2 à 10% en poids d'un propulseur.

2. Composition selon la revendication 1, dans laquelle l'huile est une huile de triglycéride .

3. Composition selon la revendication 2, dans laquelle l'huile de triglycéride comprend un ou plusieurs groupe(s) ester à chaîne ramifiée.

4. Composition selon la revendication 3, dans laquelle l'huile de triglycéride est le tri-(2-éthylhexanoate ) de glycéryle.

5. Composition selon la revendication 1, dans laquelle l'huile est le myristate d'isopropyle.

6. Composition selon la revendication 1, dans laquelle l'huile est l'oléate de décyle.

7. Composition selon l'une quelconque des revendications précédentes, dans laquelle l'émulsifiant présente un indice d'amphipathie de 12 à 14.

8. Composition selon l'une quelconque des revendications précédentes, dans laquelle l'émulsifiant est choisi parmi :

    Laureth 2 (marque déposée),

    Laureth 7 (marque déposée),

    Laurate de PEG 8 (marque déposée),

    Pareth 25,7 (marque déposée),

    Pareth 91-6 (marque déposée),

    et

    Nonoxynol 7 (marque déposée).

9. Composition selon l'une quelconque des revendications précédentes, qui comprend en outre une huile hydrocarbonée.

10. Composition selon l'une quelconque des revendications précédentes, dans laquelle le propulseur est le CAP 30 (marque déposée).

11. Composition selon l'une quelconque des revendications précédentes, qui comprend :

    a. de 85 à 95% en poids d'un concentré comprenant:

    i. de 15 à 30% en poids de tri(2-éthylhexanoate) de glycéryle;

    ii. de 2 à 10% en poids de Laureth 7 ayant un indice d'amphipathie moyen de 12,5 à 13,5 ; et

    iii. de 20 à 70% en poids d'eau ; et

    b. de 3 à 10% en poids de CAP 30.